(19) **Europäisches Patentamt**
**European Patent Office**
**Office européen des brevets**

(11) **EP 3 127 545 B1**

(12) **EUROPEAN PATENT SPECIFICATION**

(45) Date of publication and mention
of the grant of the patent:
**03.06.2020 Bulletin 2020/23**

(21) Application number: **15774351.9**

(22) Date of filing: **03.04.2015**

(51) Int Cl.:
*A61K 31/7072* (2006.01)    *A61K 31/337* (2006.01)
*A61K 31/513* (2006.01)    *A61P 1/00* (2006.01)
*A61P 11/00* (2006.01)    *A61P 15/00* (2006.01)
*A61P 35/00* (2006.01)    *A61P 43/00* (2006.01)

(86) International application number:
**PCT/JP2015/060645**

(87) International publication number:
**WO 2015/152409 (08.10.2015 Gazette 2015/40)**

(54) **ANTI-TUMOR DRUG CONTAINING TAXANE COMPOUND, AND ANTI-TUMOR EFFECT ENHANCER**

ANTITUMORWIRKSTOFF MIT TAXANVERBINDUNG UND ANTITUMORWIRKUNGSVERSTÄRKER

MÉDICAMENT ANTI-TUMORAL CONTENANT UN COMPOSÉ TAXANE, ET RENFORÇATEUR D'EFFET ANTI-TUMORAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.04.2014 JP 2014078243**

(43) Date of publication of application:
**08.02.2017 Bulletin 2017/06**

(73) Proprietor: **Taiho Pharmaceutical Co., Ltd.**
**Chiyoda-ku**
**Tokyo 101-8444 (JP)**

(72) Inventor: **OKABE, Hiroyuki**
**Tsukuba-shi**
**Ibaraki 300-2611 (JP)**

(74) Representative: **Plougmann Vingtoft a/s**
**Strandvejen 70**
**2900 Hellerup (DK)**

(56) References cited:
**WO-A1-2011/069962      US-A1- 2006 167 031**
**US-A1- 2014 066 385**

- **T. UTSUGI: "New Challenges and Inspired Answers for Anticancer Drug Discovery and Development", JAPANESE JOURNAL OF CLINICAL ONCOLOGY., vol. 43, no. 10, 1 October 2013 (2013-10-01), pages 945-953, XP055326467, JP ISSN: 0368-2811, DOI: 10.1093/jjco/hyt131**
- **I.V. BIJNSDORP ET AL.: 'Synergistic interaction between trifluorothymidine and docetaxel is sequence dependent' CANCER SCI. vol. 99, no. 11, 18 October 2008, ISSN 1432-1335 pages 2302 - 2308, XP019938581**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to an anti-tumor agent comprising a combination drug of trifluridine and tipiracil hydrochloride and a taxane compound and to an anti-tumor effect enhancer for a taxane compound.

BACKGROUND ART

Trifluridine (another name: $\alpha,\alpha,\alpha$-trifluorothymidine; hereinafter also called "FTD") interferes with DNA synthesis by inhibition of thymidylate synthesis and interferes with DNA function by incorporation into DNA, thus exerting anti-tumor effects. Meanwhile, tipiracil hydrochloride (chemical name: 5-chloro-6-[(2-iminopyrrolidin-1-yl)methyl]pyrimidine-2,4(1 H, 3H)-dione hydrochloride; hereinafter also called "TPI") has a thymidine phosphorylase inhibitory effect. It is known that TPI suppresses in vivo degradation of FTD by thymidine phosphorylase, thus enhancing the anti-tumor effect of FTD (Patent Literature 1). At the present time, an anti-tumor agent comprising FTD and TPI at a molar ratio of 1:0.5 (hereinafter also called "FTD·TPI combination drug") has been developed as a therapeutic agent of solid cancers and is approved in Japan as a therapeutic agent for advanced or recurrent colorectal cancer (Non-Patent Literature 1 and 2).
In order to enhance the anti-tumor effect of the FTD·TPI combination drug, combination therapies have been studied, and the studies have suggested combination effects of the combination drug or FTD with irinotecan, oxaliplatin, docetaxel, or the like (Non-Patent Literature 3 to 5).
Non-Patent Literature 5 discloses the synergy between Docetaxel and Trifluridine and suggests the potential for combining TAS-102 with Docetaxel. Non-Patent Literature 5 contains no disclosure of the combination of triflupiridine, tipiracil and paclitaxel being actually administered to a patient.
Taxane compounds are anti-tumor agents that stabilize microtubules in cells and thereby inhibit cell division, thus exerting anti-tumor effects. Taxane compounds such as paclitaxel and docetaxel are clinically used against a wide variety of cancer types including breast cancer, gastric cancer, and non-small cell lung cancer. It is reported that taxane compounds can be used in combination with many anti-tumor agents such as carboplatin (Non-Patent Literature 6).

CITATION LIST

PATENT LITERATURE

Patent Literature 1: International Publication WO 96/30346

NON-PATENT LITERATURE

Non-Patent Literature 1: Invest New Drugs 26 (5): 445-54, 2008.
Non-Patent Literature 2: Lancet Oncol. 13 (10): 993-1001, 2012.
Non-Patent Literature 3: Eur J Cancer. 43 (1): 175-83, 2007.
Non-Patent Literature 4: Br J Cancer. 96 (2) : 231-40, 2007.
Non-Patent Literature 5: Cancer Sci. 99 (11): 2302-8, 2008.
Non-Patent Literature 6: Curr Clin Pharmacol. 5 (3) : 226-31, 2010.

SUMMARY OF INVENTION

TECHNICAL PROBLEM

The present invention has an object to provide a novel cancer treatment composition comprising a FTD·TPI combination drug that exhibits markedly excellent anti-tumor effects with fewer side effects.

SOLUTION TO PROBLEM

The present inventor has found that a FTD·TPI combination drug, which exhibits markedly excellent anti-tumor effects with acceptable side effects when used alone, surprisingly exhibits markedly enhanced anti-tumor effects without serious side effects when the FTD·TPI combination drug is used in combination with a taxane compound (especially paclitaxel), as compared with the case where either the FTD·TPI combination drug or the taxane compound is used alone.

Non-Patent Literature 6 discloses a combination use of FTD (TFT in Non-Patent Literature 6) and docetaxel but merely discloses in vitro combination tests, and there is no study about side effects. Hence, Non-Patent Literature 6 does not disclose whether the FTD·TPI combination drug and docetaxel can be actually administered in combination so as to exert anti-tumor effects with side effects being suppressed. In addition, Non-Patent Literature 6 does not disclose or suggest the preferred concentration ranges described in the present invention.

In other words, the present invention relates to the following aspects.

[1] An anti-tumor agent characterized in that
a taxane compound and a combination drug containing trifluridine and tipiracil hydrochloride at a molar ratio of 1:0.5 are administered in combination.

[2] The anti-tumor agent according to the above [1], wherein a daily dose of the combination drug on an administration day of the combination drug is 50 to 100% of a recommended dose of the combination drug for use in monotherapy, and a daily dose of the taxane compound on an administration day of the taxane compound is 25 to 100% of a recommended dose of the taxane compound for use in monotherapy.

[3] The anti-tumor agent according to the above [1] or [2], wherein the taxane compound is paclitaxel.

[4] The anti-tumor agent according to any one of the above [1] to [3], wherein a daily dose of the combination drug on an administration day of the combination drug is 35 to 70 mg/m$^2$/day.

[5] The anti-tumor agent according to any one of the above [1] to [4], wherein a daily dose of paclitaxel on an administration day of paclitaxel is 105 to 210 mg/m$^2$/day on a once-every-three-week schedule.

[6] The anti-tumor agent according to any one of the above [1] to [4], wherein a daily dose of paclitaxel on an administration day of paclitaxel is 50 to 100 mg/m$^2$/day on a once-every-week schedule.

[7] The anti-tumor agent according to any one of the above [1] to [6], wherein a target cancer is digestive cancer.

[8] The anti-tumor agent according to any one of the above [1] to [6], wherein a target cancer is gastric cancer.

[9] An anti-tumor effect enhancer for enhancing an anti-tumor effect of a taxane compound, the anti-tumor effect enhancer consisting of a combination drug containing trifluridine and tipiracil hydrochloride at a molar ratio of 1:0.5.

[10] An anti-tumor effect enhancer for enhancing an anti-tumor effect of a combination drug containing trifluridine and tipiracil hydrochloride at a molar ratio of 1:0.5, the anti-tumor effect enhancer consisting of a taxane compound.

[11] An anti-tumor agent for treating a cancer patient having received a taxane compound, the anti-tumor agent consisting of a combination drug containing trifluridine and tipiracil hydrochloride at a molar ratio of 1:0.5.

[12] An anti-tumor agent for treating a cancer patient having received a combination drug containing trifluridine and tipiracil hydrochloride at a molar ratio of 1:0.5, the anti-tumor agent consisting of a taxane compound.

[13] An anti-tumor agent consisting of a combination drug containing trifluridine and tipiracil hydrochloride at a molar ratio of 1:0.5, the anti-tumor agent being used in combination with a taxane compound.

[14] An anti-tumor agent consisting of a taxane compound, the anti-tumor agent being used in combination with a combination drug containing trifluridine and tipiracil hydrochloride at a molar ratio of 1:0.5.

[28] The anti-tumor agent according to any one of the above [1] to [8] for use in treating a tumor.

[29] The anti-tumor effect enhancer according to the above [9] or [10] for use in enhancing an anti-tumor effect.

[30] An anti-tumor agent consisting of a combination drug containing trifluridine and tipiracil hydrochloride at a molar ratio of 1:0.5, for use in treating a cancer patient having received a taxane compound.

[31] An anti-tumor agent consisting of a taxane compound for use in treating a cancer patient having received a combination drug containing trifluridine and tipiracil hydrochloride at a molar ratio of 1:0.5.

ADVANTAGEOUS EFFECTS OF INVENTION

The present invention enables cancer treatment that achieves high anti-tumor effects (especially, tumor regression effect, tumor growth delay effect (life span increasing effect)) with side effects being suppressed. This enables cancer patients to live for a longer period of time.

BRIEF DESCRIPTION OF DRAWINGS

Fig. 1 is a view showing anti-tumor effects in mice having received the FTD·TPI combination drug (a molar ratio of FTD and TPI of 1:0.5) at a dose of 75 mg/kg/day in terms of FTD in combination with paclitaxel at a dose of 5 mg/kg/day.
Fig. 2 is a view showing anti-tumor effects in mice having received the FTD·TPI combination drug (a molar ratio of FTD and TPI of 1:0.5) at a dose of 75 mg/kg/day in terms of FTD in combination with paclitaxel at a dose of 20 mg/kg/day.
Fig. 3 is a view showing anti-tumor effects in mice having received the FTD·TPI combination drug (a molar ratio of FTD and TPI of 1:0.5) at a dose of 150 mg/kg/day in terms of FTD in combination with paclitaxel at a dose of 5 mg/kg/day.

Fig. 4 is a view showing anti-tumor effects in mice having received the FTD·TPI combination drug (a molar ratio of FTD and TPI of 1:0.5) at a dose of 150 mg/kg/day of in terms of FTD in combination with paclitaxel at a dose of 20 mg/kg/day.

## DESCRIPTION OF EMBODIMENTS

The present invention relates to an anti-tumor agent characterized in that a FTD·TPI combination drug and a taxane compound are administered in combination, an anti-tumor effect enhancer, and uses thereof.

FTD and TPI, which are used in the present invention, are known compounds and can be synthesized by the methods disclosed in International Publication WO 96/30346, for example. A combination drug containing FTD and TPI at a molar ratio of 1: 0.5 is also well-known (Non-Patent Literature 1 and 2). The FTD·TPI combination drug is approved in Japan as a therapeutic agent for advanced or recurrent colorectal cancer. The dosage regimen thereof is specified as follows: First, the combination drug is orally administered at a dose of 70 mg/m$^2$/day in terms of FTD twice a day for five consecutive days, and then a 2-day rest period is taken. This cycle is repeated twice, and then a 14-day rest period is taken. This is defined as one course, and the course is repeated.

The definition of the "taxane compound" in the present invention is part of common general technical knowledge, and the taxane compound may be any compound that has a taxane ring and has anti-tumor activities. The taxane compound is specifically exemplified by paclitaxel, docetaxel, and cabazitaxel. Of them, preferred is paclitaxel.

Paclitaxel (chemical name: (-)-(1S,2S,3R,4S,5R,7S,8S,10R,13S)-4,10-diacetoxy-2-benzoyl oxy-5,20-epoxy-1,7-dihydroxy-9-oxotax-11-en-13-yl(2R,3S)-3-benzoylamino-2-hydroxy-3-phenylpropionate) is a known compound and can be synthesized by the method disclosed in International Publication WO 99/45001. Its commercial product (Taxol inj. (registered trademark), Bristol-Myers) can also be used.

Docetaxel (chemical name: (2R,3S)-N-carboxy-3-phenylisoserine, N-tert-butyl ester, 13-ester with 5, 20-epoxy-1,2,4,7,10,13-hexahydroxytax-11-en-9-one 4-acetate 2-benzoate, trihydrate) is a known compound and can be synthesized by the method disclosed in JP-B No. 06-051689. Its commercial product (Taxotere for intravenous infusion (registered trademark), Sanofi) can also be used.

In the present invention, the FTD·TPI combination drug can be administered to humans and other mammals (for example, rats, mice, rabbits, sheep, pigs, cows, cats, dogs, and monkeys). In the present invention, the taxane compound can be administered to humans and other mammals (for example, rats, mice, rabbits, sheep, pigs, cows, cats, dogs, and monkeys).

In the present invention, the daily dose of the FTD·TPI combination drug (a FTD:TPI molar ratio of 1:0.5) on an administration day is preferably 50 to 100% of a recommended dose of the FTD·TPI combination drug for use in monotherapy, and more preferably 100%, in view of the enhancement of the anti-tumor effects of a taxane compound by the FTD·TPI combination drug. Specifically, the recommended dose of the FTD·TPI combination drug for use in monotherapy in humans is 70 mg/m$^2$/day in terms of FTD, which is the dose approved in Japan as mentioned above. Accordingly, the daily dose of the FTD·TPI combination drug on an administration day is preferably 35 to 70 mg/m$^2$/day and more preferably 70 mg/m$^2$/day in terms of FTD in the present invention.

In the present invention, the "recommended dose" is a dose that is determined by clinical trials or the like and provides a maximum therapeutic effect within such a safety range as not to cause serious side effects. Specifically, the recommended dose includes doses that are approved, recommended, or advised by public institutions or groups including Japanese Pharmaceuticals and Medical Devices Agency (PMDA), U.S Food and Drug Administration (FDA), and European Medicines Agency (EMA) and are described on package inserts, interview forms, treatment guidelines, or the like. The recommended dose is preferably a dose approved by a public institution selected from PMDA, FDA, and EMA.

In the present invention, the daily dose of the taxane compound on an administration day is preferably 50 to 100% of a recommended dose of the taxane compound for use in monotherapy, and more preferably 100%, in view of the enhancement of the anti-tumor effects of the taxane compound by the FTD·TPI combination drug. Specifically, in accordance with the approved information on a package insert or an interview form, the recommended dose of the taxane compound such as paclitaxel for use in monotherapy in humans on a once-every-three-week schedule (for example, head and neck cancer, digestive cancer, non-small cell lung cancer, breast cancer, and endometrial cancer; preferably non-small cell lung cancer, gastric cancer, endometrial cancer, and breast cancer) is 210 mg/m$^2$/day, and the recommended dose of the taxane compound for use in monotherapy on a once-every-week schedule (for example, head and neck cancer, digestive cancer, non-small cell lung cancer, breast cancer, and endometrial cancer; preferably head and neck cancer, esophageal cancer, and breast cancer) is 100 mg/m$^2$/day. Accordingly, the daily dose of the taxane compound such as paclitaxel on an administration day is preferably 105 to 210 mg/m$^2$/day and more preferably 210 mg/m$^2$/day on a once-every-three-week schedule, and the daily dose is preferably 50 to 100 mg/m$^2$/day and more preferably 100 mg/m$^2$/day on a once-every-week schedule.

The administration schedule of the anti-tumor agent of the present invention can be appropriately determined depending on cancer types and disease stages, for example. For humans, the FTD·TPI combination drug is preferably administered on such an administration schedule that 5-day consecutive administration and a 2-day rest period are repeated twice and followed by a 2-week rest period or on such an administration schedule that 5-day consecutive administration and a 9-day rest period are repeated twice. For humans, the taxane compound such as paclitaxel is preferably administered on a once-every-three-week schedule or on a once-every-week schedule. The above-described administration schedule may be repeated. A rest period may be provided depending on side effects and the like.

The daily administration frequency of the anti-tumor agent of the present invention can be appropriately determined depending on cancer types and disease stages, for example. The FTD·TPI combination drug is preferably administered twice a day, and the taxane compound such as paclitaxel is preferably administered once a day.

The order of the administration of the FTD·TPI combination drug and the taxane compound such as paclitaxel of the present invention can be appropriately determined depending on cancer types and disease stages, for example. Either one can be administered earlier, or both can be administered simultaneously.

The target cancer of the present invention is specifically exemplified by digestive cancer (for example, esophageal cancer, gastric cancer, duodenal cancer, liver cancer, biliary tract cancer (including gallbladder cancer and bile duct cancer), pancreatic cancer, small intestinal cancer, and colorectal cancer (including colorectal cancer, colon cancer, and rectal cancer)). Here, the cancer includes not only primary tumors but also metastatic cancers in other organs (for example, the liver) . Of them, in view of anti-tumor effects and side effects, the target cancers are preferably digestive cancer and gastric cancer. The anti-tumor agent of the present invention may be used for postoperative adjuvant chemotherapy for recurrence prevention after surgical removal of tumors or used for preoperative adjuvant chemotherapy performed before surgical removal of tumors.

The administration schedules of the active ingredients are different, and hence the active ingredients cannot be mixed and formulated into a single preparation. The anti-tumor agent of the present invention is thus prepared in such a way that the active ingredients are formulated into a plurality of preparations. In other words, FTD and TPI are preferably formulated as a combination preparation, and the taxane compound such as paclitaxel is preferably formulated as a single preparation.

The plurality of preparations may be packed and sold in a single package suited for combination administration, or in separate packages as long as the active ingredients are administered at their respective doses specified in the present invention.

The dosage form of the anti-tumor agent of the present invention is not limited to particular forms and can be appropriately selected according to the therapeutic purpose. The dosage form is specifically exemplified by oral preparations (including tablets, coated tablets, powders, granules, capsules, and liquids), injections, suppositories, adhesive patches, and ointments. The combination drug of FTD and TPI is preferably prepared as an oral preparation. The taxane compound such as paclitaxel can be prepared in any of the above dosage forms and is preferably prepared in the form of an injection.

For the anti-tumor agent in the present invention, the FTD·TPI combination drug and the single drug of the taxane compound such as paclitaxel can be prepared with the use of pharmaceutically acceptable carriers according to a known method appropriate for the dosage form. Such a carrier is exemplified by general purpose carriers commonly used in medicinal agents, such as excipients, binders, disintegrants, lubricants, diluents, solubilizing agents, suspending agents, tonicity agents, pH adjusters, buffers, stabilizers, coloring agents, flavoring agents, and odor improving agents.

The present invention also relates to an anti-tumor effect enhancer comprising a FTD·TPI combination drug for enhancing the anti-tumor effect of a taxane compound in cancer patients (especially, gastric cancer patients). The anti-tumor effect enhancer can be prepared as any of the dosage forms mentioned for the above anti-tumor agent.

The present invention also relates to an anti-tumor effect enhancer comprising a taxane compound for enhancing the anti-tumor effect of a FTD·TPI combination drug in cancer patients (especially, gastric cancer patients). The anti-tumor effect enhancer can be prepared as any of the dosage forms mentioned for the above anti-tumor agent.

The present invention also relates to an anti-tumor agent comprising a FTD·TPI combination drug, for treating cancer patients (especially, gastric cancer patients) having received a taxane compound. The anti-tumor agent can be prepared as any of the dosage forms mentioned above.

The present invention also relates to an anti-tumor agent comprising a taxane compound, for treating cancer patients (especially, gastric cancer patients) having received a FTD·TPI combination drug. The anti-tumor agent can be prepared as any of the dosage forms mentioned above.

The present invention also relates to an anti-tumor agent comprising a FTD·TPI combination drug, and the anti-tumor agent is administered in combination with a taxane compound to cancer patients (especially, gastric cancer patients). The anti-tumor agent can be prepared as any of the dosage forms mentioned above.

The present invention also relates to an anti-tumor agent comprising a taxane compound, and the anti-tumor agent is administered in combination with a FTD·TPI combination drug to cancer patients (especially, gastric cancer patients). The anti-tumor agent can be prepared as any of the dosage forms mentioned above.

The present invention also relates to the anti-tumor agent of the present invention for use in treating a tumor.

The present invention also relates to the anti-tumor effect enhancer of the present invention for use in enhancing an anti-tumor effect.

The present invention also relates to an anti-tumor agent consisting of a combination drug containing trifluridine and tipiracil hydrochloride at a molar ratio of 1:0.5, for use in treating a cancer patient having received a taxane compound.

The present invention also relates to an anti-tumor agent consisting of a taxane compound for use in treating a cancer patient having received a combination drug containing trifluridine and tipiracil hydrochloride at a molar ratio of 1:0.5.

EXAMPLES

The present invention will next be described in more detail with reference to examples and reference examples.

Reference Example 1

Cultured cells ($1 \times 10^7$ cells/mouse) of a human colon cancer cell line (KM20C) were transplanted into the abdominal cavity of 5 to 6-week-old BALB/cA Jcl-nu mice. The mice were divided into groups (n = 10) in such a way that each group had an equal mean body weight, and the grouping day was defined as Day 0.

A FTD·TPI combination drug (a mixture of FTD and TPI at a molar ratio of 1:0.5) was prepared for administration at doses of 75, 100, 150, 300, and 450 mg/kg/day in terms of FTD. The administration of the drug was started from Day 3. The FTD·TPI combination drug was orally administered for five consecutive days and then a 2-day rest period was taken. This cycle was repeated for 6 weeks.

As an index of the anti-tumor effect, the number of living mice was counted in each group, and the life spans and the increased life spans of the groups were compared. The increased life span (ILS) was calculated in accordance with the following equation.

```
ILS (%) = [{(mean life span of administered group)/(mean

life span of control group)} - 1] × 100
```

Table 1 shows the results.

Table 1

| Group | Dose (mg/kg/day in terms of FTD) | Mean life span (day) Mean $\pm$ SD | ILS (%) |
|---|---|---|---|
| Control | - | 40.0 $\pm$ 4.3 | - |
| FTD·TPI combination | 75 | 50.0 $\pm$ 9.1 | 25.0 |
| FTD·TPI combination | 100 | 75.8 $\pm$ 42.6 | 89.5 |
| FTD·TPI combination | 150 | 125.7 $\pm$ 64.8 | 214.3 |
| FTD·TPI combination | 300 | 75.6 $\pm$ 17.5 | 89.0 |
| FTD·TPI combination | 450 | 54.1 $\pm$ 18.3 | 35.3 |

As shown in Table 1, the FTD·TPI combination drug showed life span increasing effect in all the groups at 75 to 450 mg/kg/day in terms of FTD. Of them, the group at 150 mg/kg/day showed a maximum life span. Thus, the recommended dose (RD) of the FTD·TPI combination drug for mice is 150 mg/kg/day in terms of FTD. In other words, the results indicate that the FTD·TPI combination drug exerts the life span increasing effect at least at a dose of 50% to 300% of the RD.

Meanwhile, it is known that the RD of the FTD·TPI combination drug for humans is 70 mg/m²/day in terms of FTD. Accordingly, as for the dose of the FTD·TPI combination drug in terms of FTD, 150 mg/kg/day for mice corresponds to 70 mg/m²/day for humans.

Reference Example 2

A human gastric cancer cell line (SC-2) was transplanted to the right chest of 5 to 6-week-old BALB/cA Jcl-nu mice. After the tumor transplantation, the long diameter (mm) and the short diameter (mm) of the tumor were measured, and the tumor volume (TV) was calculated. The mice were divided into groups (n = 6) in such a way that each group had an equal mean TV, and the grouping day was defined as Day 0. Paclitaxel was administered at doses of 10, 20, and 30 mg/kg/day on Days 1 and 8. As a result, after the second administration at 30 mg/kg/day, four of the six mice deceased. The recommended dose of paclitaxel for mice was thus 20 mg/kg/day.

Example: Combination use of FTD·TPI combination drug and paclitaxel on SC-2

A human gastric cancer cell line (SC-2) was transplanted to the right chest of 5 to 6-week-old BALB/cA Jcl-nu mice. After the tumor transplantation, the long diameter (mm) and the short diameter (mm) of the tumor were measured, and the tumor volume (TV) was calculated. The mice were divided into groups (n = 6) in such a way that each group had an equal mean TV, and the grouping day was defined as Day 0.

The FTD·TPI combination drug (a mixture of FTD and TPI at a molar ratio of 1:0.5) was prepared for administration at a dose of 150 mg/kg/day in terms of FTD. Paclitaxel (TXL, Wako Pure Chemical Industries, Ltd.) was prepared for administration at doses of 5 and 20 mg/kg/day. The FTD·TPI combination drug was orally administered on Days 1 to 14 consecutively, and paclitaxel was administered through the tail vein on Day 1 and Day 8. In the combination administration groups, the doses and administration schedules of the FTD·TPI combination drug and paclitaxel were the same as those of the corresponding monotherapy groups.

As an index of the anti-tumor effect, the TVs on Days 3, 7, 12, 15, 19, 22, 26, and 29 were calculated in each group. In accordance with the following equation, the relative tumor volume (RTV) to that on Day 0 was calculated and compared with the RTV of the untreated group (control). The combination effect was evaluated as follows: when the mean RTV value of a combination administration group is statistically, significantly smaller (closed testing procedure; intersection-union test, $p < 0.01$) than those of the corresponding monotherapy groups, the combination administration was regarded as having an enhancement effect. The results are shown in Table.

$$\text{TV (mm}^3) = (\text{long diameter} \times \text{short diameter}^2)/2$$

$$\text{RTV} = (\text{TV on Day 29})/(\text{TV on Day 0})$$

The RTVs were plotted on the indicated measurement days, and RTV changes over days in the control group, the FTD·TPI combination drug administration group, the paclitaxel administration group, and the combination administration group of the FTD·TPI combination drug and paclitaxel were compared.

The tumor growth inhibition rate (IR) on Day 29 on the basis of RTV values was calculated in accordance with the following equation.

$$\text{IR (\%)} = [1 - (\text{mean RTV value of treated group})/(\text{mean RTV value of control group})] \times 100$$

The results of the control group and the single-drug administration groups on Day 29 are shown in Table 2.

To evaluate body weight reduction as an index of side effects, body weights were measured in each group on each measurement day.

Table 2

| Drug | Dose (mg/kg/day) | RTV[a] (mean ± SD) | IR[b] (%) |
|---|---|---|---|
| Control | - | 61.73 ± 8.78 | - |
| FTD·TPI combination | 75 | 31.80 ± 4.18** | 48.5 |
| FTD·TPI combination | 150 | 20.24 ± 2.23** | 67.2 |
| Paclitaxel (TXL) | 5 | 44.97 ± 1.78** | 27.1 |
| Paclitaxel (TXL) | 20 | 10.13 ± 2.94** | 83.6 |
| FTD·TPI combination + TXL | 75 + 5 | 16.64 ± 0.98 **## | 73.0 |
| FTD·TPI combination + TXL | 75 + 20 | 2.70 ± 0.50 **## | 95.6 |

(continued)

| Drug | Dose (mg/kg/day) | RTV[a] (mean $\pm$ SD) | IR[b] (%) |
|---|---|---|---|
| FTD·TPI combination + TXL | 150 + 5 | 13.77 $\pm$ 1.16 **## | 77.7 |
| FTD·TPI combination + TXL | 150 + 20 | 1.47 $\pm$ 0.65 **## | 97.6 |

**: $p < 0.01$ vs. Control by Dunnett's test.
##: overall maximal $p < 0.01$ by closed testing procedure (Intersection-Union Test), respectively.

a) : Relative tumor volume (RTV) on Day 29 was calculated as the ratio of TV on Day 29 to that on Day 0 according to the following formula:

$$RTV = (TV\ on\ Day\ 29)/(TV\ on\ Day\ 0)$$

b): Tumor growth inhibition rate (IR) on Day 29 on the basis of RTV was calculated according to the following formula:

$$IR\ (\%) = [1 - (mean\ RTV\ of\ the\ treated\ group)/(mean\ RTV\ of\ the\ control\ group)] \times 100$$

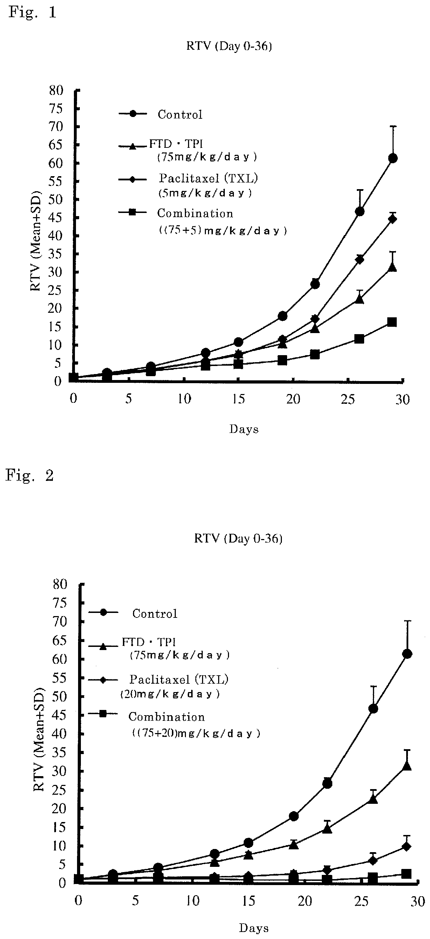

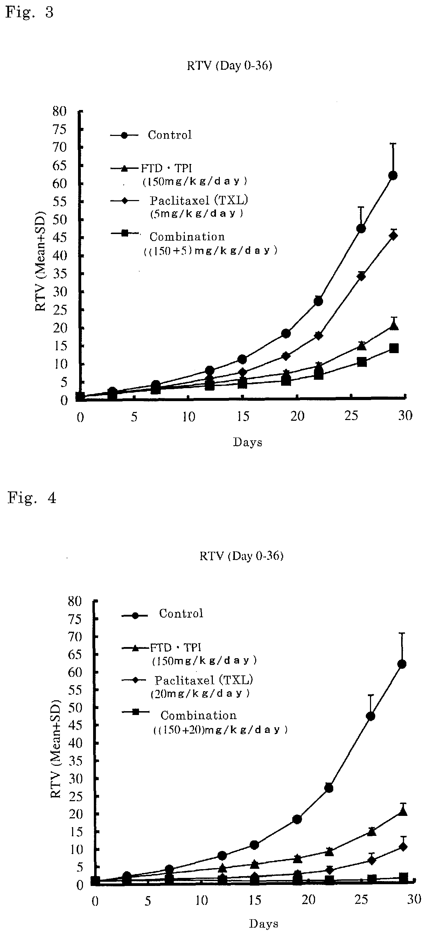

As shown in Table 2 and Figs. 1 to 4, statistically significantly enhanced anti-tumor effects were observed when the FTD·TPI combination drug was administered at 75 to 150 mg/kg/day in terms of FTD and paclitaxel was administered at 5 to 20 mg/kg/day. In the combination administration groups of the FTD·TPI combination drug and paclitaxel, no serious body weight reduction (more than 20% reduction) was observed, and side effects were acceptable.

The above results show that the anti-tumor effect is significantly enhanced with side effects being suppressed when the FTD·TPI combination drug is administered at 75 to 150 mg/kg/day in terms of FTD and paclitaxel is administered at 5 to 20 mg/kg/day. As shown in Reference Examples, the recommended dose of the FTD·TPI combination drug for use in monotherapy is 150 mg/kg/day (in terms of FTD), and the recommended dose of paclitaxel for use in monotherapy is 20 mg/kg/day. This indicates that a marked enhancement of the anti-tumor effect was observed when the FTD·TPI combination drug was administered at 50 to 100% of the recommended dose for use in monotherapy and paclitaxel was administered at 25 to 100% of the recommended dose for use in monotherapy. As shown in Reference Example 1, it is ascertained that the life span increasing effect in the FTD·TPI combination drug administration groups at 300 to 450 mg/kg/day exceeded that in the group at 75 mg/kg/day, at which an enhancement of the anti-tumor effect by combination administration with the taxane compound was observed as shown in the above example. It is thus suggested that a significant enhancement effect on the anti-tumor effect is exerted when the FTD·TPI combination drug is administered at 75 to 450 mg/kg/day (corresponding to 50 to 300% of the recommended dose for use in monotherapy) in combination with the taxane compound at 50 to 100% of the recommended dose for use in monotherapy.

## Claims

1. An anti-tumor agent consisting of a combination drug containing trifluridine and tipiracil hydrochloride at a molar ratio of 1:0.5,
   wherein the anti-tumor agent being used in combination with a taxane compound,
   wherein a daily dose of the combination drug on an administration day of the combination drug is 50 to 100% of a recommended dose of the combination drug for use in monotherapy, and a daily dose of the taxane compound on an administration day of the taxane compound is 25 to 100% of a recommended dose of the taxane compound for use in monotherapy,
   wherein the taxane compound is paclitaxel,
   for use in treating a tumor.

2. The anti-tumor agent for use according to claim 1, wherein a daily dose of the combination drug on an administration day of the combination drug is 35 to 70 mg/m$^2$/day.

3. The anti-tumor agent for use according to any one of claims 1 to 2, wherein a daily dose of paclitaxel on an administration day of paclitaxel is 105 to 210 mg/m$^2$/day on a once-every-three-week schedule.

4. The anti-tumor agent for use according to any one of claims 1 to 2, wherein a daily dose of paclitaxel on an administration day of paclitaxel is 50 to 100 mg/m$^2$/day on a once-every-week schedule.

5. The anti-tumor agent for use according to any one of claims 1 to 4, wherein a target cancer is digestive cancer.

6. The anti-tumor agent for use according to any one of claims 1 to 4, wherein a target cancer is gastric cancer.

7. An anti-tumor effect enhancer consisting of a combination drug containing trifluridine and tipiracil hydrochloride at a molar ratio of 1:0.5 for use in:

(i) enhancing an anti-tumor effect of a taxane compound or
(ii) treating a cancer patient having received a taxane compound,

wherein a daily dose of the combination drug on an administration day of the combination drug is 50 to 100% of a recommended dose of the combination drug for use in monotherapy, and a daily dose of the taxane compound on an administration day of the taxane compound is 25 to 100% of a recommended dose of the taxane compound for use in monotherapy,
wherein the taxane compound is paclitaxel.

8. An anti-tumor effect enhancer consisting of a taxane compound for use in:

(i) enhancing an anti-tumor effect of a combination drug containing trifluridine and tipiracil hydrochloride at a molar ratio of 1:0.5 or
(ii) treating a cancer patient having received a combination drug containing trifluridine and tipiracil hydrochloride at a molar ratio of 1:0.5,

wherein a daily dose of the combination drug on an administration day of the combination drug is 50 to 100% of a recommended dose of the combination drug for use in monotherapy, and a daily dose of the taxane compound on an administration day of the taxane compound is 25 to 100% of a recommended dose of the taxane compound for use in monotherapy,
wherein the taxane compound is paclitaxel.

9. An anti-tumor agent for use in treating a cancer patient having received a taxane compound, the anti-tumor agent consisting of a combination drug containing trifluridine and tipiracil hydrochloride at a molar ratio of 1:0.5,
wherein a daily dose of the combination drug on an administration day of the combination drug is 50 to 100% of a recommended dose of the combination drug for use in monotherapy, and a daily dose of the taxane compound on an administration day of the taxane compound is 25 to 100% of a recommended dose of the taxane compound for use in monotherapy,
wherein the taxane compound is paclitaxel.

**Patentansprüche**

1. Antitumormittel, bestehend aus einem Kombinationsarzneimittel, das Trifluridin und Tipiracilhydrochlorid in einem Molverhältnis von 1:0,5 enthält,
wobei das Antitumormittel in Kombination mit einer Taxanverbindung verwendet wird,
wobei eine tägliche Dosis des Kombinationsarzneimittels an einem Verabreichungstag des Kombinationsarzneimittels 50 bis 100 % einer empfohlenen Dosis des Kombinationsarzneimittels zur Verwendung in einer Monotherapie beträgt und eine tägliche Dosis der Taxanverbindung an einem Verabreichungstag der Taxanverbindung 25 bis 100 % einer empfohlenen Dosis der Taxanverbindung zur Verwendung in einer Monotherapie beträgt,
wobei die Taxanverbindung Paclitaxel ist,
zur Verwendung beim Behandeln eines Tumors.

2. Antitumormittel zur Verwendung nach Anspruch 1, wobei eine tägliche Dosis des Kombinationsarzneimittels an einem Verabreichungstag des Kombinationsarzneimittels 35 bis 70 mg/m$^2$/Tag beträgt.

3. Antitumormittel zur Verwendung nach einem der Ansprüche 1 bis 2, wobei eine tägliche Dosis von Paclitaxel an einem Verabreichungstag von Paclitaxel 105 bis 210 mg/m$^2$/Tag in einem Einmal-alle-drei-Wochen-Plan beträgt.

4. Antitumormittel zur Verwendung nach einem der Ansprüche 1 bis 2, wobei eine tägliche Dosis von Paclitaxel an einem Verabreichungstag von Paclitaxel 50 bis 100 mg/m$^2$/Tag in einem Einmal-jede-Woche-Plan beträgt.

5. Antitumormittel zur Verwendung nach einem der Ansprüche 1 bis 4, wobei ein Zielkrebs ein Krebs des Verdauungstrakts ist.

6. Antitumormittel zur Verwendung nach einem der Ansprüche 1 bis 4, wobei ein Zielkrebs Magenkrebs ist.

7. Antitumorwirkungsverstärker, bestehend aus einem Kombinationsarzneimittel, das Trifluridin und Tipiracilhydrochlorid in einem Molverhältnis von 1:0,5 enthält, zur Verwendung bei:

   (i) Verstärken einer Antitumorwirkung einer Taxanverbindung oder
   (ii) Behandeln eines Krebspatienten, der eine Taxanverbindung erhalten hat,

   wobei eine tägliche Dosis des Kombinationsarzneimittels an einem Verabreichungstag des Kombinationsarzneimittels 50 bis 100 % einer empfohlenen Dosis des Kombinationsarzneimittels zur Verwendung in einer Monotherapie beträgt und eine tägliche Dosis der Taxanverbindung an einem Verabreichungstag der Taxanverbindung 25 bis 100 % einer empfohlenen Dosis der Taxanverbindung zur Verwendung in einer Monotherapie beträgt,
   wobei die Taxanverbindung Paclitaxel ist.

8. Antitumorwirkungsverstärker, bestehend aus einer Taxanverbindung, zur Verwendung bei:

   (i) Verstärken einer Antitumorwirkung eines Kombinationsarzneimittels, das Trifluridin und Tipiracilhydrochlorid in einem Molverhältnis von 1:0,5 enthält, oder
   (ii) Behandeln eines Krebspatienten, der ein Kombinationsarzneimittel erhalten hat, das Trifluridin und Tipiracilhydrochlorid in einem Molverhältnis von 1:0,5 enthält,

   wobei eine tägliche Dosis des Kombinationsarzneimittels an einem Verabreichungstag des Kombinationsarzneimittels 50 bis 100 % einer empfohlenen Dosis des Kombinationsarzneimittels zur Verwendung in einer Monotherapie beträgt und eine tägliche Dosis der Taxanverbindung an einem Verabreichungstag der Taxanverbindung 25 bis 100 % einer empfohlenen Dosis der Taxanverbindung zur Verwendung in einer Monotherapie beträgt,
   wobei die Taxanverbindung Paclitaxel ist.

9. Antitumormittel zur Verwendung beim Behandeln eines Krebspatienten, der eine Taxanverbindung erhalten hat, das Antitumormittel bestehend aus einem Kombinationsarzneimittel, das Trifluridin und Tipiracilhydrochlorid in einem Molverhältnis von 1:0,5 enthält,
   wobei eine tägliche Dosis des Kombinationsarzneimittels an einem Verabreichungstag des Kombinationsarzneimittels 50 bis 100 % einer empfohlenen Dosis des Kombinationsarzneimittels zur Verwendung in einer Monotherapie beträgt und eine tägliche Dosis der Taxanverbindung an einem Verabreichungstag der Taxanverbindung 25 bis 100 % einer empfohlenen Dosis der Taxanverbindung zur Verwendung in einer Monotherapie beträgt,
   wobei die Taxanverbindung Paclitaxel ist.

**Revendications**

1. Agent antitumoral composé d'une association de médicaments contenant de la trifluridine et du chlorhydrate de tipiracil à un rapport molaire de 1:0,5,
   dans lequel l'agent antitumoral étant utilisé en combinaison avec un composé taxane,
   dans lequel une dose quotidienne de l'association de médicaments sur un jour d'administration de l'association de médicaments va de 50 à 100 % d'une dose recommandée de l'association de médicaments pour utilisation en monothérapie, et une dose quotidienne du composé taxane sur un jour d'administration du composé taxane va de 25 à 100 % d'une dose recommandée du composé taxane pour utilisation en monothérapie,
   dans lequel le composé taxane est le paclitaxel, pour une utilisation dans le traitement d'une tumeur.

2. Agent antitumoral pour utilisation selon la revendication 1, dans lequel une dose quotidienne de l'association de médicaments sur un jour d'administration de l'association de médicaments va de 35 à 70 mg/m$^2$/jour.

3. Agent antitumoral pour utilisation selon l'une quelconque des revendications 1 à 2, dans lequel une dose quotidienne de paclitaxel sur un jour d'administration de paclitaxel va de 105 à 210 mg/m$^2$/jour en fonction d'un planning d'une fois toutes les trois semaines.

4. Agent antitumoral pour utilisation selon l'une quelconque des revendications 1 à 2, dans lequel une dose quotidienne de paclitaxel sur un jour d'administration de paclitaxel va de 50 à 100 mg/m$^2$/jour en fonction d'un planning d'une fois par semaine.

5. Agent antitumoral pour utilisation selon l'une quelconque des revendications 1 à 4, dans lequel un cancer cible est le cancer digestif.

6. Agent antitumoral pour utilisation selon l'une quelconque des revendications 1 à 4, dans lequel un cancer cible est le cancer gastrique.

7. Amplificateur de l'effet antitumoral composé d'une association de médicaments contenant de la trifluridine et du chlorhydrate de tipiracil à un rapport molaire de 1:0,5 pour une utilisation dans :

   (i) l'amplification d'un effet antitumoral d'un composé taxane ou
   (ii) le traitement d'un patient atteint d'un cancer ayant reçu un composé taxane,

   dans lequel une dose quotidienne de l'association de médicaments sur un jour d'administration de l'association de médicaments va de 50 à 100 % d'une dose recommandée de l'association de médicaments pour utilisation en monothérapie, et une dose quotidienne du composé taxane sur un jour d'administration du composé taxane va de 25 à 100 % d'une dose recommandée du composé taxane pour utilisation en monothérapie,
   dans lequel le composé taxane est le paclitaxel.

8. Amplificateur de l'effet antitumoral composé d'un composé taxane pour une utilisation dans :

   (i) l'amplification d'un effet antitumoral d'une association de médicaments contenant de la trifluridine et du chlorhydrate de tipiracil à un rapport molaire de 1:0,5 ou
   (ii) le traitement d'un patient atteint d'un cancer ayant reçu une association de médicaments contenant de la trifluridine et du chlorhydrate de tipiracil à un rapport molaire de 1:0,5,

   dans lequel une dose quotidienne de l'association de médicaments sur un jour d'administration de l'association de médicaments va de 50 à 100 % d'une dose recommandée de l'association de médicaments pour utilisation en monothérapie, et une dose quotidienne du composé taxane sur un jour d'administration du composé taxane va de 25 à 100 % d'une dose recommandée du composé taxane pour utilisation en monothérapie,
   dans lequel le composé taxane est le paclitaxel.

9. Agent antitumoral pour utilisation dans le traitement d'un patient atteint d'un cancer ayant reçu un composé taxane, l'agent antitumoral étant composé d'une association de médicaments contenant de la trifluridine et du chlorhydrate de tipiracil à un rapport molaire de 1:0,5,
   dans lequel une dose quotidienne de l'association de médicaments sur un jour d'administration de l'association de médicaments va de 50 à 100 % d'une dose recommandée de l'association de médicaments pour utilisation en monothérapie, et une dose quotidienne du composé taxane sur un jour d'administration du composé taxane va de 25 à 100 % d'une dose recommandée du composé taxane pour utilisation en monothérapie,
   dans lequel le composé taxane est le paclitaxel.

Fig. 1

RTV (Day 0-36)

Fig. 2

RTV (Day 0-36)

Fig. 3

RTV (Day 0-36)

Fig. 4

RTV (Day 0-36)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9630346 A
- WO 9945001 A
- JP 6051689 B

**Non-patent literature cited in the description**

- *Invest New Drugs,* 2008, vol. 26 (5), 445-54
- *Lancet Oncol.,* 2012, vol. 13 (10), 993-1001
- *Eur J Cancer.,* 2007, vol. 43 (1), 175-83
- *Br J Cancer.,* 2007, vol. 96 (2), 231-40
- *Cancer Sci.,* 2008, vol. 99 (11), 2302-8
- *Curr Clin Pharmacol.,* 2010, vol. 5 (3), 226-31